# NEW EUROPEAN PATENT SPECIFICATION

(11) **EP 0 812 172 B2**
(45) Date of publication and mention of the opposition decision: **21.09.2005**
(45) Mention of the grant of the patent: 31.10.2001
(21) Application number: 96905469.1
(22) Date of filing: 14.02.1996
(51) Int. Cl.: A61F 13/15

(54) **DIAPER HAVING PLURAL UPSTANDING LEG CUFFS**
WINDEL MIT MEHREREN AUFRECHTEN BEINKLAPPEN
COUCHES A PLUSIEURS BRACELETS EN SAILLIE

(30) Priority: 01.03.1995 US 396949
(43) Date of publication of application: 17.12.1997
(73) Proprietor: THE PROCTER & GAMBLE COMPANY, Cincinnati, Ohio 45202 (US)
(72) Inventor: ROE, Donald, Carroll, West Chester, OH 45069 (US); DREIER, Kimberly, Ann, Cincinnati, OH 45251 (US)
(74) Representative: McGregor, Judit Ester
(86) International application number: PCT/US1996/001901
(87) International publication number: WO 1996/026698

(56) References cited:
- EP-A- 0 251 332
- EP-A- 0 491 390
- EP-A- 0 508 477
- EP-A- 0 549 988
- FR-A- 2 680 316
- GB-A- 2 216 393
- JP-A- 174 845
- US-A- 4 846 823

## Description

### FIELD OF THE INVENTION

This invention is related to disposable absorbent articles, particularly to disposable absorbent articles such as diapers, which receive fecal material, and more particularly to diapers having upstanding leg cuffs.

### BACKGROUND OF THE INVENTION

Disposable absorbent articles, such as diapers, are well known in the art. These articles address the consumers' demands for increased convenience. In particular, disposable absorbent articles which minimize cleaning of the wearer after the article is soiled provide convenience. A particularly desired feature in such diapers is the prevention or minimization of leakage of urine and fecal material received by the diaper.

Several attempts in the art have been very successful at reducing leakage from the diaper. One early attempt provided an elastic contracting member, which elastically contracted the outer side portion of the diaper. This highly successful advance in the art provided a gasket leg cuff. The gasket leg cuff was disposed in the plane of the diaper. The next attempt provided a diaper having elasticized flaps, known as barrier leg cuffs. Barrier leg cuffs stand up out of the plane of the diaper and thereby improve containment. Yet other attempts in the art included diapers with dual cuffs, including both a gasket leg cuff and a barrier leg cuff. Yet other advances in the art provided leg cuffs with relatively low contact forces against the skin of the wearer at relatively high elongations, minimizing wearer discomfort.

Examples of such attempts in the art can be found in commonly assigned U.S. Patents 3,860,003 issued January 14, 1975 to Buell; 4,695,278 issued September 22, 1987 to Lawson; 4,909,803 issued March 20, 1990 to Aziz et al.; and 5,032,120 issued July 18, 1991 to Freeland et al.

Another example can be found In EP-A-0 491 390 and JP-H2-174845. This diaper defines the preamble of Claim 1.

However, there is a need in the art for diapers which further reduce leakage. There is further a need in the art for such a diaper which minimizes leakage of fecal material from the leg region.

### BRIEF DESCRIPTION OF THE DRAWINGS

Figure 1 is a top plan view, shown partially in cutaway, of a diaper according to the present invention.
Figure 2 is an instantaneous vertical sectional view taken along lines 2-2 of Figure 1.

It is not shown in this figure that the outer barrier leg cuff 32 has a greater extent in the Z-direction than the inner barrier leg cuff 30.

### SUMMARY OF THE INVENTION

The invention comprises a diaper having a longitudinal centerline and a lateral centerline orthogonal to the longitudinal centerline. The diaper has a liquid pervious topsheet, a liquid impermeable backsheet at least partially peripherally joined to the topsheet, and an absorbent core intermediate the topsheet and the back-sheet.

Upstanding from the plane of the topsheet are two generally longitudinally oriented liquid impervious outer barrier leg cuffs. One of the outer barrier leg cuffs is disposed on each side of the longitudinal centerline. Also upstanding from the plane of the topsheet are two generally longitudinally oriented liquid pervious inner barrier leg cuffs. One of each of the innerbarrier leg cuffs is also disposed on each side of the longitudinal centerline. Each of the inner barrier leg cuffs is disposed between the corresponding outer barrier leg cuff and the longitudinal centerline. Each of the outer barrier leg cuffs is outboard of and preferably spaced apart from the corresponding inner barrier leg cuff 1.27 cm to 5.08 cm (0.5 to 2.0 inches).

The diaper has a Z-direction perpendicular to said longitudinal centerline and said lateral centerline. The outer barrier leg cuffs have a greater extent in the Z-direction than the inner barrier leg cuffs and the outer barrier leg cuffs and inner barrier leg cuffs have specific elastic forces as defined in claim 1.

### DETAILED DESCRIPTION OF THE INVENTION

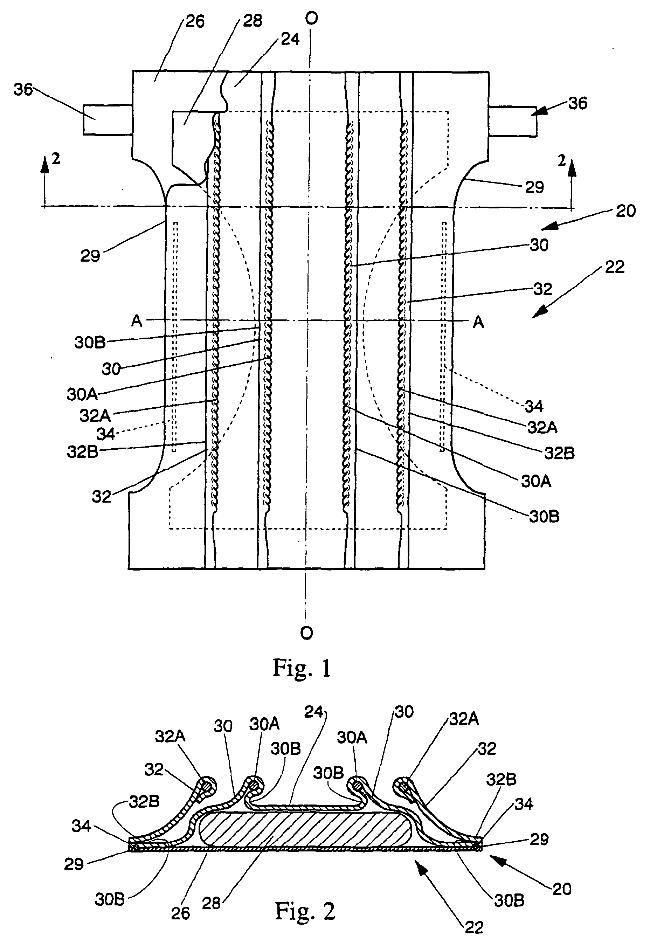

As used herein, the term "absorbent article" refers to devices which absorb and contain body exudates and are placed against or in proximity to the body of the wearer to absorb and contain discharges. The term "disposable" describes absorbent articles not intended to be laundered or otherwise restored or reused (i.e., they are intended to be discarded after a single use and preferably recycled, composted, or otherwise disposed of in an environmentally compatible manner). A "unitary" absorbent article refers to an article formed of separate parts united together to form a coordinated entity that does not require separate manipulative parts, like a separate holder and liner. A preferred embodiment of a disposable absorbent article of the present invasion is the unitary disposable absorbent article, diaper 20, shown in Figure 1. As used herein the term "diaper" refers to a disposable absorbent article generally worn by infants and incontinent persons about the lower torso. It should be understood, however, that the present invention is also applicable to other disposable absorbent articles such as incontinence briefs, incontinence undergarments, and diaper holders and liners.

Figure 1 is a plan view of diaper 20 of the present invention in its flat, uncontracted state (with elastic induced contraction pulled out, and portions of the structure cut away to more clearly show the construction of the diaper 20). The portion of the diaper 20 which faces or contacts the wearer, i.e., the inner surface, is oriented towards the viewer. The diaper 20 has a longitudinal centerline O-O and a lateral centerline A-A. As used here in the longitudinal centerline O-O or dimension is aligned front to back and bisects the standing wearer into left and right body halves. The lateral centerline A-A or dimension is orthogonal the longitudinal centerline O-O and lies within the plane of the diaper 20. The Z-direction is orthogonal to both the longitudinal and lateral directions and comes out of the plane of the diaper 20.

The diaper 20 has a chassis 22 comprising a liquid pervious topsheet 24, a liquid impermeable backsheet 26 at least partially peripherally joined to the topsheet 24, and an absorbent core 28 between the topsheet 24 and the backsheet 26. The topsheet 24 has an inwardly oriented surface which is oriented towards the core 28, and an outwardly oriented surface which is oriented towards and/or contacts the wearer. Upstanding from the plane of the topsheet 24 are a set of inner barrier leg cuffs 30, and a set of outer barrier leg cuffs 32. Outboard of the outer barrier leg cuffs 32 and in line within the plane of the diaper 20 are gasket cuffs 34. As used herein, "outboard" refers to either lateral direction which is oriented away from the longitudinal centerline O-O.

The diaper 20 may also include tape fasteners 36 positioned in the rear waist region for fastening the diaper 20 about the wearer. The diaper 20 can also have an elastic waistband (not shown). Commonly assigned U.S. Patents 3,848,594 issued November 19, 1974 to Buell; Re B1 4,662,875 issued May 5, 1987 to Hirotsu et al. are to illustrate tape fasteners 36; and 4,515,959 issued May 17, 1985 to Kiev; and 4,816,025 issued March 28, 1989 to Foreman, are to illustrate elasticized waist features.

The topsheet 24 and backsheet 26 of the diaper 20 have longitudinal and lateral dimensions generally larger than those of the absorbent core 28, so that the topsheet 24 and backsheet 26 may extend beyond the core 28 to thereby form the periphery 29 of the diaper 20. The embodiment described herein is suitable for a wearer weighing about 7.3 to about 12.7 kilograms (16 to 28 pounds). It will be understood that if the diaper 20 is intended for use with larger or smaller wearers, including adults, or if the diaper 20 is closed in a training pants style, the diaper 20, including the inner and outer barrier leg cuffs 30, 32, may have to be scaled accordingly.

Examining the components of the diaper 20 in more detail, the topsheet 24 and backsheet 26 are generally coextensive and at least partially peripherally joined. As used herein, the term "joined" refers to the condition where a first member or component is affixed or connected to a second member or component, either directly or indirectly where the first member or component is directly affixed to the second member or component, or connected to an intermediate member or component which in turn is affixed or connected to the second member or component. Components which are "joined" are intended to remain affixed or connected throughout the intended life of the diaper 20 and not to be separated unless and until the diaper 20 is discarded and as may be necessary for environmentally compatible disposal. Components which are "joined" cannot be separated without tearing or gross deformation of one or both components.

The topsheet 24 refers to any liquid pervious facing of the diaper 20 which contacts the skin of the wearer and prevents substantial contact of the absorbent core 28 with the skin of the wearer. The topsheet 24 is compliant, tactilely pleasant, and non-irritating to the skin.

A suitable topsheet 24 may be manufactured from porous foams, apertured plastic films, natural fibers, synthetic fibers, or a combination thereof. A particularly preferred topsheet 24 comprises polypropylene fibers and may be manufactured as a nonwoven web of spunbonded, carded, wet laid, melt blown, hydroentangled fibers. A particularly preferred topsheet 24 is carded and thermally bonded to have a basis weight of 14 to 25 grams per square meter. A suitable topsheet 24 is marketed by Veratec Inc., Division of International Paper Company, of Walpole, Massachusetts under the designation P-8.

The backsheet 26 is impermeable to fluids such as urine and prevents fluids absorbed and contained by the core 28 from wetting the undergarments. As used herein, the "backsheet" refers to any barrier disposed outwardly of the core 28 as the diaper 20 is worn and which contains absorbed liquid within the diaper 20. The backsheet 26 is preferably manufactured from a thin plastic film, although other flexible, liquid impermeable materials may be used. As used herein, the term "flexible" refers to materials which are compliant and will readily conform to the general shape of the human body.

The backsheet 26 may be a polyolefinic film, such as polyethylene, having a thickness of about 0.01 to 0.05 millimeters. A suitable backsheet 26 can be made from a blend of 45 to 90 percent LLDP and about 10 to 55 percent polypropylene. Exemplary backsheet films are sold by Tredegar Industries of Terre Haute, Indiana under the designation RR8220 and RR5475.

The topsheet 24 and backsheet 26 may be joined by any means well known in the art, such as adhesive bonding or heat sealing. A particularly preferred method of joining the topsheet 24 and backsheet 26 is with hot melt adhesives such as are manufactured by Century Adhesives, Inc. of Columbus, Ohio and marketed as Century 5227, or BL1258 adhesive sold by the H. B. Fuller Company of St. Paul, Minnesota, or H2031 available from the Findley Adhesives Company of Elmgrove, Wisconsin.

As used herein, the term "core" refers to any component of the diaper 20 intermediate the topsheet 24 and backsheet 26 and used for absorbing and retaining body exudates. The core 28 may be encased by one or more layers of tissue (not shown).

The absorbent core 28 may be made from a variety of materials such as comminuted wood pulp and may further contain particulate or fibrous absorbent gelling materials as are commonly known in the art. The absorbent core 28 may be made in accordance with the teachings of commonly assigned U.S. Patents 4,610,678 issued September 9, 1986 to Weisman et al.; 5,137,537 issued August 11, 1992 to Herron et al.; and 5,147,345 issued September 15, 1992 to Young et al. Absorbent gelling materials, if desired, may be made in accordance with commonly assigned U.S. Patent Re 32,649, reissued April 19, 1988 to Brandt et al.

Referring to Figure 2, the inner barrier leg cuffs 30 may, in part, overlie the core 28 and are generally longitudinally oriented. Preferably each inner barrier leg cuff 30 is laterally spaced a distance of 2.0 to 3.5 inches (5,08-8,89 cm) from the other inner barrier leg cuff 30. Such spacing is taken at the proximal edges 30B of the inner barrier leg cuffs 30. Generally, for the embodiment described herein, this spacing is critical for proper performance of the diaper 20. Such a spacing is necessary to allow sufficient room for urine and fecal material to be deposited on the topsheet 24 at a position intermediate the inner barrier leg cuffs 30. Therefore, the inner barrier leg cuffs 30 should not be spaced any closer together than this range. Likewise, the inner barrier leg cuffs 30 should not be spaced too far apart, otherwise the inner barrier leg cuffs 30 will be too close to the outer barrier leg cuffs 32, as discussed below.

The inner barrier leg cuffs 30 are preferably elastically contractible due to elastic strands 30A joined to the inner barrier leg cuffs 30. The elastic strands 30A may be made using any type of elastic as is well known in the art. Of course, elastic strands 30A, 32A include elastomeric films, elastomeric adhesives, as well as elastic strands, and combinations thereof. The inner barrier leg cuffs 30 may have an elasticized length in the longitudinal direction of about 8 to 15 inches. The elastic strands 30 may have a force of 10 to 30 grams (0.098 to 0.294 Newton), and preferably 15 to 25 grams (0.147 to 0.245 Newton), at 85 percent elongation. Preferably the inner barrier leg cuff 30 extends 0.5 to 1.25 inches (1.27 to 3.175 cm) above the plane of the topsheet 24 to a distal edge.

The inner barrier leg cuffs 30 are preferably hydrophilic and liquid pervious. These material properties are critical in that they allow urine deposited on the topsheet 24 to laterally migrate through the inner barrier leg cuffs 30 so that full utilization of the capacity of the absorbent core 28 is possible. Conversely, if the inner barrier leg cuffs 30 were liquid impervious, urine would pool between the inner barrier leg cuffs 30, irritating the skin of the wearer and may lead to premature leakage. An additional benefit of a hydrophilic pervious inner barrier leg cuff 30 is that low viscosity fecal material which contacts the inner barrier leg cuff 30 may be partitioned into solid components which are contained between the inner barrier leg cuffs 30 and liquid components which permeate the inner barrier leg cuffs 30. By partitioning the low viscosity fecal material in this manner, the likelihood of it leaking is reduced.

Preferably the inner barrier leg cuffs 30 are made of a nonwoven material, as is available from the Veratec Company of Walpole, Massachusetts. The inner barrier leg cuffs 30 may be adhesively joined to the topsheet 24 as is well known in the art. The distal end of the inner barrier leg cuff 30 may be joined to the topsheet 24 at the waist margins of the diaper 20, as shown. The upstanding portion of the inner barrier leg cuff 30 may be liquid impervious, in part, so long as urine can pass through at least a portion thereof, and the proximal edge 30B does not prevent urine from passing into the topsheet 24.

Each outer barrier leg cuff 32 is preferably spaced 0.5 to 2.0 inches (1.27 to 5.08 cm) laterally outboard (i.e., away from the longitudinal centerline O-O) of its corresponding inner barrier leg cuff 30. Such spacing is taken at the proximal edge 32B of the outer barrier leg cuff 32. Inner barrier leg cuffs 30, outer barrier leg cuffs 32, and gasket cuffs 34 are said to be "corresponding" if they are disposed on the same side of the longitudinal centerline O-O.

It is, of course, recognized that the inner and outer barrier leg cuffs 30, 32 may not always be straight and parallel, as shown. Instead, one set of barrier leg cuffs 30, 32 may be longitudinally oriented as shown, and the other set of barrier leg cuffs 32, 30 be in angular relationship therewith. For purposes of the present invention, the spacing criterion is met if at least 33 percent of the upstanding portions of the inner and outer barrier leg cuffs 30, 32 fall within the aforementioned range of 0.5 to 2.0 inches (1.27 to 5.08 cm). Although a minimum of 50 percent effective spacing is preferred, at least 75 percent is more preferred. Preferably, the aforementioned spacing is met at the rear portion of the diaper 20 because this is most typically where fecal material is loaded. This spacing between the inner and outer barrier leg cuffs 30, 32 is critical. If the inner and outer barrier leg cuffs 30, 32 are spaced too close together, they will function as a single barrier leg cuff and not be effective at reducing leakage. Likewise, the distal ends of the inner and outer barrier leg cuffs 30, 32 must be separated, and not joined together, otherwise they will function like a single leg cuff. Conversely, the outer barrier leg cuff 32 cannot be spaced further from the inner barrier leg cuff 30 than the constraints imposed by the periphery 29 of the diaper 20. As noted above, once the periphery 29 of the diaper 20 is fixed, the inner barrier leg cuffs 30 (as measured between the proximal edges 30) cannot be moved closer together, otherwise the urine and fecal material may be deposited on the inner barrier leg cuffs 30, or between the inner and outer barrier leg cuffs 30, 32. If either occurs, leakage is more likely to result.

The outer barrier leg cuffs 32 are preferably elastically contractible due to elastic strands 32A joined to the outer barrier leg cuffs 32. The elastic strands 32A may be made using any type of elastic as is well known in the art. The outer barrier leg cuffs 32 may have an elasticized length taken in the longitudinal direction of about 8 to 15 inches (20.32 to 38.1 cm), and have a force of 30 to 65 grams, and preferably 40 to 50 grams at 85 percent elongation.

It is important that the outer barrier leg cuffs 32 have a greater contractive force under a given elongation than the inner barrier leg cuffs 30, so that a tighter seal is formed at the leg of the wearer. Additionally, the inner leg cuffs 30 typically intercept the skin of the wearer at a position of the body more prone to red marking, and the discomfort attendant therewith. Therefore, less force should be applied by the inner barrier leg cuff 30 than the outer barrier leg cuff 32.

Preferably the outer barrier leg cuffs 32 extend 0.75 to 2.0 inches (1.905 to 5.08 cm) above the plane of the topsheet 24. The outer barrier leg cuffs 32 preferably extend further in the Z-direction than the inner barrier leg cuffs 30, so that overflow past the inner barrier leg cuffs 30 does not reach a second barrier leg cuff 32 of the same height and allow leakage. Also, this greater Z-direction extent is necessary to accommodate the curvature of the buttocks of the wearer.

It is critical that the outer barrier leg cuff 32 be hydrophobic and/or liquid impervious in order that any urine, low viscosity fecal material, or components of low viscosity fecal material which permeated the inner barrier leg cuff 30 is retained inboard (i.e., towards the longitudinal centerline O-O) of the outer barrier leg cuffs 32 so that leakage does not result. The outer barrier leg cuffs 32 may be made of a nonwoven material such as is available from Fiberweb Company of Simpsonville, South Carolina.

Optionally, a gasket cuff 34 is included in the diaper 20. The gasket cuff 34 may be spaced inboard or outboard of the outer barrier leg cuff 32 so long as the gasket cuff 34 is outboard of the inner barrier leg cuff 30. Preferably the gasket cuff 34 is outboard of the outer barrier leg cuff 32, so that the periphery 29 of the diaper 20 more readily conforms to the shape of the buttocks, and so that it is spaced far enough from the absorbent core 28 to be effective. In an alternative embodiment (not shown), the gasket cuff 34 may be positioned between the inner barrier leg cuff 30 and the outer barrier leg cuff 32. While the present invention does allow for bringing the gasket cuff 34 to within less than 0.75 inches (1.905 cm) of the edge of the absorbent core 28, contrary to important teachings in the prior art, such a spacing is generally not preferred.

## Claims

1. A diaper (20) having a longitudinal centerline (O-O) and a lateral centerline (A-A) orthogonal thereto, said diaper (20) comprising:
a liquid pervious topsheet (24);
a liquid impervious backsheet (26) at least partially peripherally joined to said topsheet (24), an absorbent core (28) intermediate said topsheet (24) and said backsheet (26);
two generally longitudinally oriented outer barrier leg cuffs (32), upstanding from the plane of said topsheet (24), one of said outer barrier leg cuffs (32) being disposed on each side of said longitudinal centerline; and
two generally longitudinally oriented inner barrier leg cuffs (30) upstanding from the plane of said topsheet (24), one of said inner barrier leg cuffs (30) being disposed on each side of said longitudinal centerline, so that each said inner barrier leg cuff (30) is disposed on the same side of said longitudinal centerline as one of said outer barrier leg cuffs (32) corresponding thereto, each said corresponding outer barrier leg cuff (32) and said corresponding inner barrier leg cuff (30) being spaced apart 1.27 cm to 5.08 cm (0.5 inches to 2.0 inches), said diaper (20) having a Z-direction perpendicular to said longitudinal centerline and said lateral centerline, and said outer barrier leg cuffs (32) have a greater extent in the Z-direction than said inner barrier leg cuffs (30), **characterized in that** said inner barrier leg cuffs (30) have an elastic force at 85 percent elongation of 0.147 to 0.245 Newton (15 to 25 grams), and **in that** said outer barrier leg cuffs (32) have an elastic force at 85% elongation of 0.392 Newton to 0.637 Newton (40 to 65 grams).

2. A diaper (20) according to Claim 1 **characterized in that** said inner barrier leg cuffs (30) are spaced apart 5.08 to 8.89 cm (2.0 to 3.5 inches).

3. A diaper (20) according to Claim 1 wherein said outer barrier leg cuffs (32) are liquid impervious and said inner barrier leg cuffs (30) are liquid pervious.

4. A diaper (20) according to Claims 1, 2 or 3, wherein said outer barrier leg cuffs (32) are hydrophobic, and more preferably said inner barrier leg cuffs (30) are hydrophilic.

## Patentansprüche

1. Windel (20) mit einer längs verlaufenden Mittellinie (O-O) und einer quer verlaufenden Mittellinie (A-A) rechtwinklig dazu, wobei die Windel (20) umfasst:
eine flüssigkeitsdurchlässige Oberschicht (24);
eine flüssigkeitsundurchlässige Unterschicht (26), die wenigstens teilweise umfänglich mit der Oberschicht (24) verbunden ist, einen absorbierenden Kern (28) zwischen der Oberschicht (24) und der Unterschicht (26);
zwei im Wesentlichen längs ausgerichtete, innere Barrieren-Beinaufschläge (32), die von der Ebene der Oberschicht (24) nach oben abstehen, wobei einer der inneren Barrieren-Beinaufschläge (32) auf jeder Seite der längs verlaufenden Mittellinie angeordnet ist; und
zwei im Wesentlichen längs ausgerichtete, innere Barrieren-Beinaufschläge (30), die von der Ebene der Oberschicht (24) nach oben abstehen, wobei einer der inneren Barrieren-Beinaufschläge (30) auf der gleichen Seite der längs verlaufenden Mittellinie wie einer der damit korrespondierenden äußeren Barrieren-Beinaufschläge (32) angeordnet ist, wobei der korrespndierende äußere Barrieren-Beinaufschlag (32) und der korrespondierende innere Barrieren-Beinaufschlag (30) in einem Abstand von 1,27 cm (0,5 Zoll) bis 5,08 cm (2,0 Zoll) zueinander liegen, wobei die Windel (20) eine Z-Richtung senkrecht zur längs verlaufenden Mittellinie und zur quer verlaufenden Mittellinie hat, **dadurch gekennzeichnet, dass** die äußeren Barrieren-Beinaufschläge (32) eine größere Ausdehnung in Z-Richtung haben als die inneren Barrieren-Beinaufschläge (30), **dadurch gekennzeichnet, dass** die inneren Barrieren-Beinaufschläge (30) eine elastische Kraft von 0,147 Newton (15 bis 25 Gramm) bei einer Verlängerung von 85% aufweisen, und **dadurch**, dass die äußeren Barrieren-Beinaufschläge (32) eine elastische Kraft von 0,392 Newton bis 0,637 Newton (40 bis 65 Gramm) bei einer Verlängerung von 85% aufweisen.

2. Windel (20) nach Anspruch 1 **dadurch gekennzeichnet, dass** die inneren Barrieren-Beinaufschläge (30) in einem Abstand von 5.08 bis 8.89 cm (2.0 bis 3.5 Zoll) zueinander liegen.

3. Windel (20) nach Anspruch 1, in welcher die äußeren Barrieren-Beinaufschläge (32) flüssigkeitsundurchlässig sind und die inneren Barrieren-Beinaufschläge (30) flüssigkeitsundurchlässig sind.

4. Windel (20) nach Anspruch 1, 2 or 3, in welcher die äußeren Barrieren-Beinaufschläge (32) hydrophob sind, und mehr bevorzugt die inneren Barrieren-Beinaufschläge (30) hydrophob sind.

## Revendications

1. Couche (20) présentant une ligne médiane longitudinale (O-O) et une ligne médiane latérale (A-A) orthogonale à celle-ci, ladite couche (20) comprenant:
une feuille de dessus perméable aux liquides (24);
une feuille de fond imperméable aux liquides (26) réunie au moins partiellement de manière périphérique à ladite feuille de dessus (24), une âme absorbante (28) placée entre ladite feuille de dessus (24) et ladite feuille de fond (26);
deux revers de jambe de barrière extérieurs orientés globalement longitudinalement (32), situés perpendiculairement au plan de ladite feuille de dessus (24), un desdits revers de jambe de barrière extérieurs (32) étant placé sur chaque côté de ladite ligne médiane longitudinale; et
deux revers de jambe de barrière intérieurs (30) orientés globalement longitudinalement, situés perpendiculairement au plan de ladite feuille de dessus (24), un desdits revers de jambe de barrière intérieurs (30) étant placé sur chaque côté de ladite ligne médiane longitudinale, afin que chaque dit revers de jambe de barrière intérieur (30) soit placé sur le même côté de ladite ligne médiane longitudinale qu'un desdits revers de jambe de barrière extérieurs (32) correspondant à ceux-ci, chaque dit revers de jambe de barrière extérieur correspondant (32) et dit revers de jambe de barrière intérieur correspondant (30) étant espacé de 1,27 cm à 5,08 cm (0,5 pouce à 2,0 pouces), ladite couche (20) ayant une direction Z perpendiculaire à ladite ligne médiane longitudinale et ladite ligne médiane latérale, et lesdits revers de jambe de barrière extérieurs (32) ont une plus grande étendue dans la direction Z que lesdits revers de jambe de barrière intérieurs (30), **caractérisée en ce que** lesdits revers de jambe de barrière intérieurs (30) ont une force élastique, pour un allongement de 85 pour-cent, de 0,147 à 0,245 Newton (15 à 25 grammes), et **en ce que** lesdits revers de jambe de barrière extérieurs (32) ont une force élastique, pour un allongement de 85 %, de 0,392 Newton à 0,637 Newton (40 à 65 grammes).

2. Couche (20) selon la revendication 1, **caractérisée en ce que** lesdits revers de jambe de barrière intérieurs (30) sont espacés de 5,08 à 8,89 cm (2,0 à 3,5 pouces).

3. Couche (20) selon la revendication 1, dans laquelle lesdits revers de jambe de barrière extérieurs (32) sont imperméables aux liquides et lesdits revers de jambe de barrière intérieurs (30) sont perméables aux liquides.

4. Couche (20) selon les revendications 1, 2 ou 3, dans laquelle lesdits revers de jambe de barrière extérieurs (32) sont hydrophobes et, mieux encore, lesdits revers de jambe de barrière intérieurs (30) sont hydrophiles.
